(19) Europäisches Patentamt

European Patent Office

Office européen des brevets

(11) Veröffentlichungsnummer: **0 251 303 B1**

(12) **EUROPÄISCHE PATENTSCHRIFT**

(45) Veröffentlichungstag der Patentschrift: **26.08.92**

(21) Anmeldenummer: **87109423.1**

(22) Anmeldetag: **30.06.87**

(51) Int. Cl.⁵: **A61K 31/19**, //(A61K31/19, 31:045)

Die Akte enthält technische Angaben, die nach dem Eingang der Anmeldung eingereicht wurden und die nicht in dieser Patentschrift enthalten sind.

(54) **Viruzides Mittel mit Breitbandwirkung.**

(30) Priorität: **02.07.86 DE 3622089**

(43) Veröffentlichungstag der Anmeldung:
**07.01.88 Patentblatt 88/01**

(45) Bekanntmachung des Hinweises auf die Patenterteilung:
**26.08.92 Patentblatt 92/35**

(84) Benannte Vertragsstaaten:
**AT BE CH DE ES FR GB GR IT LI LU NL SE**

(56) Entgegenhaltungen:
**FR-A- 2 490 928**

**DIALOG 5048609, EXERPTA MEDICA 82049978; S.A. SPROSSIG et al.:
"Virusdesinfektion - Anforderungen und Moglichkeiten", & HYG. MED. (GERMANY, WEST), 1981, 6/10 (449-453)**

**CHEMICAL ABSTRACTS, Band 70, Nr. 13, 31. März 1969, Seite 121, Nr. 55176n, Columbus, Ohio, US; M. SPROESSIG et al.:
"Antimicrobial activity of peracetic acid. V. Virucidal effect", & PHARMAZIE 1968, 23(11), 665-7**

**Pharmazie 1968 23(11) 665-7**

(73) Patentinhaber: **Krüger GmbH & Co. KG
Meisenweg 2 Postfach 20 05 29
W-5060 Bergisch Gladbach 2(DE)**

(72) Erfinder: **Höffler, Jutta. Dr.
Woldsenweg 7
W-2000 Hamburg 20(DE)**
Erfinder: **Eggers, Hans-J., Prof.-Dr.
Fürst-Pückler-Strasse 56
W-5000 Köln 41(DE)**

(74) Vertreter: **Werner, Hans-Karsten, Dr. et al
Deichmannhaus am Hauptbahnhof
W-5000 Köln 1(DE)**

## Beschreibung

Gegenstand der vorliegenden Erfindung ist ein viruzides Mittel mit Breitbandwirkung, inbesondere gegen verschiedenste Arten von nackten Viren, nämlich den als besonders problematisch geltenden Virusstämmen Poliovirus 1 (Stamm Mahoney), Vacciniavirus (Stamm Elstree), SV 40-Virus (z.B. Stamm 777) und Adenovirus 2 (Stamm Adenoid 6).

Es ist bekannt, daß Viren mit einer Lipoidhülle relativ empfindlich sind und daher auch mit bisher bekannten viruziden Desinfektionsmitteln inaktiviert werden können. Problematisch und wesentlich beständiger gegen übliche Desinfektionsmittel sind hingegen die nackten Viren, die sich bisher eigentlich nur mit relativ hohen Konzentrationen von Formaldehyd inaktivieren lassen. Formaldehyd ist jedoch aus toxikologischen Gründen unerwünscht und gestattet weder in der Klinik noch im Labor die Desinfektion von kontaminierten Körperteilen.

Die Literatur über die desinfizierende bzw. über die inaktivierende Wirksamkeit von handelsüblichen, hautverträglichen Desinfektionsmitteln ist widersprüchlich. Auch die Angaben über die Wirksamkeit von Alkoholen sind widersprüchlich. Untersuchungen über die Wirksamkeit von Isopropanol gegen nackte Viren haben beispielsweise gezeigt, daß dieser Alkohol nur sehr schwach wirksam oder unwirksam ist. Diese Untersuchungen haben weiterhin ergeben, daß Ethanol und Methanol hoch wirksam sind, wenn sie weniger als 30% Wasser enthalten. Aus der DE-OS 34 30 709 der Anmelderin ist daher ein viruzides Mittel gegen nackte Viren bekannt, welches aus mindestens 70% Methanol und/oder Ethanol und 1 bis 10% Glycerin besteht.

Zusätzlich kann dieses viruzide Mittel bis zu 5% Rizinusöl enthalten. Eingehende Untersuchungen dieser viruziden Mittel auf Basis von Methanol und/oder Ethanol mit Zusätzen von Glycerin und Rizinusöl gegenüber einem breiten Spektrum von nackten Viren haben jedoch ergeben, daß diese Mittel bei einer Art von problematischen Viren versagen, nämlich dem Adenovirus 2 (Stamm Adenoid 6) und SV 40-Virus (z.B. Stamm 777).

Aus der DE-OS 32 27 126 ist bekannt, daß man die Ausbreitung respiratorischer Viren unterbrechen oder verhindern kann, indem man die Viren mit einer kurzkettigen organischen Säure in Kontakt bringt. Als bevorzugte organische Säuren werden dort Zitronensäure, Äpfelsäure, Bernsteinsäure und Benzoesäure sowie substituierte Derivate hiervon genannt. Weiterhin wird angegeben, daß die Wirksamkeit durch oberflächenaktive Mittel verbessert werden kann, wobei das Natriumsalz von 1,4-Bis-(2-äthylhexyl)-ester von Sulfobernsteinsäure und Natriumdodecylsulfat als bevorzugt erwähnt werden. Diese Mittel sind wirksam gegen die üblichen respiratorischen Viren wie Rhinoviren, Parainfluenzaviren und Adenoviren. Sie werden vorzugsweise eingesetzt, indem man diese Säuren gegebenenfalls zusammen mit dem Netzmittel auf Cellulosegewebe oder Textilien aufbringt. Als weitere Anwendungsformen sind Nasensprays, Gesichtscremes, Handlotionen und Lippenstifte genannt.

Aus den hierin aufgeführten Versuchsergebnissen geht hervor, daß diese säurehaltigen Mittel auch gegenüber Adenovirus 5 eine gewisse Wirksamkeit aufwiesen; jedoch wurde vielfach nur eine Reduzierung um 2 Zehnerpotenzen bzw. 99% beobachtet, und das zum Teil erst nach 5 Minuten Einwirkungszeit und/oder relativ hohen Konzentrationen.

Von der Deutschen Vereinigung zur Bekämpfung von Viruskrankheiten und dem Bundesgesundheitsamt werden zur Zeit gemeinsam neue Vorschriften erarbeitet, wonach ein Mittel nur dann die Bezeichnung "viruzides Mittel" tragen darf, wenn es imstande ist, die folgenden vier Virusstämme in einer für den Verwendungszweck akzeptablen Zeit, z.B. für Hände in einer bis zwei Minuten, zu inaktivieren: Poliovirus 1 (Stamm Mahoney), Vacciniavirus (Stamm Elstree), SV 40-Virus (z.B. Stamm 777) und Adenovirus 2 (Stamm Adenoid 6). Es wird somit gefordert, daß als viruzide Mittel nur noch solche Mittel bezeichnet werden dürfen, die eine Breitbandwirkung aufweisen.

Aus der FR-A-2 490 928 sind bakteriostatische bzw. bakterizide Gemische bekannt, welche als aktive Komponenten Ethylalkohol und eine organische und/oder anorganische Säure oder deren Salze enthalten. Insbesondere handelt es sich um Mittel, die gegen Colibakterien wirksam sind. In den Beispielen wird mit Konzentrationen bis zu 40 % Ethylalkohol gearbeitet. Derartige Gemische sind jedoch gegenüber Viren, und insbesondere problematischen nackten Viren, völlig unwirksam.

In Sprossig et al, "Virusdesinfektion - Anforderungen und Möglichkeiten", wird über die viruzide Wirksamkeit der Peressigsäure berichtet. Die Wirksamkeit der Peressigsäure kann durch den Zusatz von Ethanol erhöht werden.

Ausführlicher wird hierüber berichtet in dem Artikel Sprossig und Mücke, Die Pharmazie (1968), 23 (11), Seiten 665 bis 667. Gerade aus diesem Artikel geht hervor, daß weder Essigsäure allein noch Alkohol noch Essigsäure und $H_2O_2$ die beobachtete stark viruzide Wirksamkeit aufweisen. Die Wirksamkeit ist somit nahezu ausschließlich auf die Peressigsäure zurückzuführen. Die Steigerung der Wirksamkeit durch Ethanol wird damit erklärt, daß die Peressigsäure besser an den Wirkungsort vordringen kann. Peressigsäure ist nur als 40 %ige Lösung stabil und in diesem Zustand schwer handzuhaben.

Viruzide Mittel enthaltend Peressigsäure sind obendrein toxisch und so aggressiv, daß sie als viruzides Hände-Desinfektionsmittel oder Desinfektionsmittel der Haut praktisch nicht in Frage kommen. Es bestand somit nach wie vor ein starkes Bedürfnis an einem viruziden Mittel mit Breitbandwirkung, welches obendrein gut hautverträglich ist.

Es wurde jetzt überraschenderweise gefunden, daß Gemische, enthaltend mindestens 70 Gew.-% Ethanol und/oder Propanole und 0,5 bis 5 Gew.-% einer Mono-, Di- und Tricarbonsäure mit einer Kettenlänge von 2 bis 4 Kohlenstoffatomen, die mit einer Hydroxylgruppe substituiert sein können oder Sulfaminsäure diese Bedingungen erfüllen und daher als viruzide Mittel mit Breitbandwirkung anzusehen sind.

Diese Ergebnisse sind besonders überraschend, da eigene Untersuchungen mit kurzkettigen organischen Säuren gezeigt hatten, daß sie beispielsweise gegen Poliovirus nahezu unwirksam sind, so daß die Ansicht der Fachleute bestätigt wurde, daß die meisten nackten Viren gegen Säuren unempfindlich sind. Offensichtlich besteht aber eine synergistische Wirkung bei den erfindungsgemäßen Gemischen aus hochprozentigem Alkohol und den speziellen kurzkettigen organischen Säuren. Langkettige organische Säuren wie Laurinsäure zeigen diese Wirkung nicht mehr. Es wird vielmehr ein ähnlicher Effekt beobachtet wie beim Zusatz von Triglyceriden zu reinem Alkohol gemäß DE-OS 34 30 709. Überraschend war auch der Befund, daß Isopropanol allein oder im Gemisch mit Ethanol und dem Zusatz von Säuren hochwirksam ist, während Isopropanol allein gemäß DE-OS 34 30 709 unwirksam ist.

Es ist somit nach wie vor unklar, worauf die Wirksamkeit des jeweiligen Alkohols und der speziellen kurzkettigen organischen Säure beruht. Beide für sich sind nur bei einigen Virusstämmen wirksam. Das erfindungsgemäß verwendete Gemisch hingegen ist in der Lage, alle als problematisch geltenden Stämme von nackten Viren ausreichend und einwandfrei zu inaktivieren.

Gegenstand der vorliegenden Erfindung ist somit die Verwendung von Gemischen enthaltend mindestens 70 Gew.-% Ethanol und/oder Propanole und 0,5 bis 5 Gew.-% einer Mono-, Di- und Tricarbonsäure mit einer Kettenlänge von 2 bis 4 Kohlenstoffatomen, die mit einer Hydroxylgruppe substituiert sein können oder Sulfaminsäure zur Herstellung eines viruziden Mittels mit Breitbandwirkung. Vorzugsweise wird diesem Mittel bis zu 5 Gew.-% eines Netzmittels zugesetzt. Um die neuen Mittel hautschonender zu machen, ohne jedoch ihre Wirksamkeit zu vermindern, ist es möglich, auch ihnen zusätzlich bis zu 5 % Glycerin und bis zu 5 % Rizinusöl zuzugeben.

Ein weiterer Gegenstand der vorliegenden Erfindung ist ein viruzides Mittel mit Breitbandwirkung enthaltend 96 Gew.-% 80 %-iges Ethanol, 2 Gew.-% Zitronensäure und 2 Gew.-% Cholindodecylsulfonat.

Die Entscheidung, ob die erfindungsgemäßen Mittel Ethanol, Isopropanol, n-Propanol oder Gemische derselben enthalten, hängt von den Kosten sowie etwaigen gesetzlichen Regelungen über den gewerblichen Gebrauch der Alkohole ab. So ist Ethanol teurer und unterliegt obendrein in vielen Ländern gesetzlichen Spezialvorschriften, die seinen Einsatz für die Herstellung der erfindungsgemäßen Mittel beeinträchtigen können. Die Wirksamkeit von Ethanol ist höher als die von Isopropanol, so daß sich aus Gründen des Preises und der Wirksamkeit auch Gemisch der beiden Alkohole anbieten können.

Die erfindungsgemäß hergestellten Mittel sind so wirksam, daß sie innerhalb von ein bis zwei Minuten die Aktivität und Infektiosität der als problematisch angesehenen Virusstämme um mindestens 4 Zehnerpotenzen reduzieren.

Bei der Anwendung der erfindungsgemäß hergestellten Mittel können diese entweder als Händedesinfektionsmittel verwendet werden oder mit Hilfe von Watte, Stofflappen oder ähnlichen Hilfsmitteln auf die infizierten Hautteile aufgebracht werden. Wegen der inaktivierenden Wirkung von Triglyceriden ist es dabei zu empfehlen, zunächst bei einer ersten Anwendung die Hautfette zu entfernen und dann das Mittel bei einer zweiten Anwendung zur Einwirkung zu bringen. Der Gehalt an Glycerin und gegebenenfalls Rizinusöl verhindert eine zu starke und unerwünschte Austrocknung der Haut und wirkt daher in ähnlicher Weise wie eine an sich erwünschte sofortige Rückfettung. Im übrigen ist es durchaus möglich, nach der Verwendung der neuen Mittel die Haut mit geeigneten, Triglycerid enthaltenden Hautpflegemitteln zu behandeln, da die geforderte Desinfektion bzw. Inaktivierung in Abwesenheit der Triglyceride erfolgt ist.

Aus den nachfolgenden Beispielen und Vergleichsversuchen geht die Herstellung, Zusammensetzung und die Wirksamkeit der erfindungsgemäßen viruziden Mittel hervor.

**Beispiel 1**

2 Gewichtsteile Zitronensäure und 2 Gewichtsteile Cholindodecylsulfonat werden in 96 Gewichtsteilen 80%-igem Ethanol gelöst. Einem Teil dieses Gemisches wurden 1 % Rizinusöl und 4 % Glycerin zugegeben.

**Beispiel 2**

2 Gewichtsteile Glycolsäure werden in 18 Gewichtsteilen Wasser und 80 Gewichtsteilen Ethanol gelöst.

## Beispiel 3

2 Gewichtsteile Zitronensäure werden in 28 Gewichtsteilen Wasser und 70 Gewichtsteilen Ethanol gelöst.

## Beispiel 4

2 Gewichtsteile Cyclohexansulfaminsäure werden in 18 Gewichtsteilen Wasser und 80 Gewichtsteilen Ethanol gelöst.

## Beispiel 5

2 Gewichtsteile Milchsäure werden in 18 Gewichtsteilen Wasser und 80 Gewichtsteilen Ethanol gelöst.

## Beispiel 6

2 Gewichtsteile Zitronensäure werden in 18 Gewichtsteilen Wasser und 80 Gewichtsteilen Isopropanol gelöst.

Die virusinaktivierende Wirkung der Gemische gemäß Beispielen 1 bis 6 wurde getestet gegenüber Poliovirus Typ 1, Stamm Mahoney in Gegenwart von 10% fötalem Kälberserum. Gemessen wurde die Reduktion des Virustiters (log 10, PBE/ml) nach Einwirkungszeiten von 1 und 2 Minuten. Es wurde gefunden, daß in allen Fällen der Virustiter um mehr als 5 Zehnerpotenzen absank. Nur bei Beispiel 6 mit Isopropanol sank der Virustiter um 4,9 Zehnerpotenzen nach 2 Minuten.

Zum Vergleich wurden gemessen 80%-iges Ethanol, 70%-iges Ethanol, 2% wässrige Glycolsäure und 2% wässrige Zitronensäure. Der Virustiter sank beim 80%-igen Ethanol um etwa 2,5 Zehnerpotenzen, beim 70%-igen Ethanol um 1,2 Zehnerpotenzen und bei den wässrigen Säuren um maximal 1/2 Zehnerpotenz.

Die Rezepturen der Beispiele l bis 6 wurden weiterhin getestet gegen Vacciniavirus (Stamm Elstree), SV 40-Virus (z.B. Stamm 777) und Adenovirus 2 (Stamm Adenoid 6). Es zeigte sich, daß in allen Fällen der Virustiter innerhalb von 1 bis 2 Minuten um mindestens 4 Zehnerpotenzen gesunken war. Ethanol oder Propanole allein zeigen keine ausreichende Wirkung bei diesen Viren.

Weitere Versuche haben ergeben, daß als kurzkettige organische Säuren vor allem Mono-, Di- und Tricarbonsäuren mit einer Kettenlänge von 2 bis 4 Kohlenstoffatomen, die mit einer Hydroxylgruppe substituiert sein können. Als Beispiele seien genannt Glycolsäure, Zitronensäure, Milchsäure, Bernsteinsäure und Äpfelsäure. Ebenfalls geeignet sind Sulfaminsäuren wie Cyclohexylsulfaminsäure.

## Patentansprüche

1. Verwendung von Gemischen enthaltend mindestens 70 Gew.-% Ethanol und/oder Propanole und 0,5 bis 5 Gew.-% einer Mono-, Di- und Tricarbonsäure mit einer Kettenlänge von 2 bis 4 Kohlenstoffatomen, die mit einer Hydroxylgruppe substituiert sein können oder Sulfaminsäure zur Herstellung eines viruziden Mittels mit Breitbandwirkung.

2. Verwendung gemäß Anspruch 1, dadurch gekennzeichnet, daß das Gemisch zusätzlich bis zu 5 Gew.-% Cholindodecylsulfonat enthält.

3. Verwendung gemäß Anspruch 1 oder 2, dadurch gekennzeichnet, daß das Gemisch als Säure Zitronensäure oder Glykolsäure enthält.

4. Verwendung gemäß einem der Ansprüche 1 bis 3, dadurch gekennzeichnet, daß es zusätzlich bis zu 5 Gew.-% Glycerin und bis zu 5 Gew.-% Rizinusöl enthält.

5. Viruzides Mittel mit Breitbandwirkung enthaltend 96 Gew.-% 80 %-iges Ethanol, 2 Gew.-% Zitronensäure und 2 Gew.-% Cholindodecylsulfonat.

6. Viruzides Mittel gemäß Anspruch 5, dadurch gekennzeichnet, daß es zusätzlich 1 Gew.-% Rizinusöl und 4 Gew.-% Glycerin enthält.

## Claims

1. Use of mixtures containing at least 70% by weight of ethanol and/or propanols and 0.5 to 5% by weight of a mono-, di- and tricarboxylic acid having a chain length of from 2 to 4 carbon atoms which may be substituted by a hydroxy group, or sulfamic acid for the preparation of a viricidal agent with broad spectrum activity.

2. The use according to claim 1, characterized in that the mixture additionally contains up to 5% by weight of choline dodecylsulfonate.

3. Use according to claim 1 or 2, characterized in that the mixture as the acid contains citric acid or glycolic acid.

4. Use according to one of the claims 1 to 3, characterized in that the mixture additionally contains up to 5% by weight of glycerol and up to 5% by weight of castor oil.

5. Viricidal agent with broad spectrum activity containing 96% by weight of 80% ethanol, 2% by weight of citric acid and 2% by weight of choline dodecylsulfonate.

6. Viricidal agent according to claim 5, characterized in that it additionally contains 1% by weight of castor oil and 4% by weight of glycerol.

**Revendications**

1. Utilisation de mélanges contenant au moins 70 % en poids d'éthanol et/ou de propanols et 0,5 à 5 % en poids d'acide mono-, di- ou tricarboxylique ayant une longueur de chaîne de 2 à 4 atomes de carbone, qui peuvent être substitués par un groupe hydroxyle, ou d'acide sulfamique, pour la préparation d'un médicament virucide à large spectre.

2. Utilisation selon la revendication 1, caractérisée en ce que le mélange contient en outre jusqu'à 5 % en poids de dodécylsulfonate de choline.

3. Utilisation selon la revendication 1 ou 2, caractérisée en ce que le mélange contient, comme acide, de l'acide citrique ou de l'acide glycolique.

4. Utilisation selon une des revendications 1 à 3, caractérisée en ce que le mélange contient en outre jusqu'à 5 % en poids de glycérol et jusqu'à 5 % en poids d'huile de ricin.

5. Médicament virucide à large spectre, contenant 96 % en poids d'éthanol à 80 %, 2 % en poids d'acide citrique et 2 % en poids de dodécylsulfonate de choline.

6. Médicament virucide selon la revendication 5, caractérisé en ce qu'il contient en outre 1 % en poids d'huile de ricin et 4 % en poids de glycérol.